# EUROPEAN PATENT APPLICATION

(11) **EP 4 438 724 A1**
(43) Date of publication of application: **02.10.2024**
(21) Application number: 23752256.0
(22) Date of filing: 31.01.2023
(51) Int. Cl.: C12N 9/10, C12N 15/54, C12N 15/63, C12N 1/15, C12N 1/19, C12N 1/21, C12P 19/28

(54) **GLYCOSIDE HYDROLASE, AND PREPARATION METHOD THEREFOR AND APPLICATION THEREOF**

(30) Priority: 14.02.2022 CN 202210132108; 30.01.2023 CN 202310045070
(71) Applicant: Shandong Henglu Biotech Co., Ltd, Jinan, Shandong 250000 (CN)
(72) Inventor: FANG, Xu, Jinan, Shandong 250004 (CN); NIU, Kangle, Jinan, Shandong 250004 (CN); HAN, Lijuan, Jinan, Shandong 250004 (CN); TANG, Qi, Jinan, Shandong 250004 (CN); JIANG, Fujiao, Jinan, Shandong 250004 (CN); HIROSE, Yoshihiko, Nagamatsu, Ogaki Gifu 503-0097 (JP)
(74) Representative: LLR
(86) International application number: PCT/CN2023/073915
(87) International publication number: WO 2023/151476

(57) **Abstract**

A glycoside hydrolase, relating to the technical field of enzyme engineering/gene engineering. Provided are an enzyme of a GH112 glycoside hydrolase family, and an application of synthesizing human milk oligosaccharides (HMOs) by using an enzyme of a GH112 glycoside hydrolase family. A synthesis reaction is catalyzed by using the enzyme of the glycoside hydrolase family 112 (GH112) that is classified according to a Carbohydrate-Active-Enzymes (CAZy) database (http://www.cazy.org); and lacto-N-tetraose (LNT) is generated in an aqueous solution comprising lactose and/or galactose and/or glactose-1-phosphate and lacto-N-triaose (LNTII). The enzyme of the glycoside hydrolase family 112 (GH112), and the preparation method therefor and the application thereof provided by the present invention have economic and social values.

## Description

### Field of the invention

The present invention relates to a glycoside hydrolase, its expression in microorganisms and its application in the synthesis of oligosaccharides, and belongs to the technical field of enzyme engineering/genetic engineering.

### Background of the invention

Human milk oligosaccharides (HMOs) are a type of complex oligosaccharides, which are composed of monosaccharides and derivatives, sialic acids and or other structural units connected through glycoside bonds. However, it took more than a hundred years for people to know it.

As early in 1886, Austrian pediatrician and microbiologist, Escherich, discovered a link between the intestinal bacteria of infants and their digestive health. In 1900, two researchers, Moro and Tissier independently corroborated that the bacterial flora in the excrement differed significantly between breast milk-fed infants and artificial milk-fed infants. In 1926, Schönfeld discovered that breast milk whey contained growth promoters for bifidobacteria. In 1930, French scientists Polonowski and Lespagnol detected a component with carbohydrate characteristics in breast milk whey, named "gynolactose". In 1954, György demonstrated that "bifidobacterium factor", a factor that promotes the growth of bifidobacteria, was actually an oligosaccharide. In the same year, Polonowski and Montreuil isolated oligosaccharides from lacto-oligosaccharides by using two-dimensional chromatography. In 1983, Egge *et al.* identified HMOs using fast atom bombardment mass spectrometry. In 1999, Coppa *et al.* detected 30-50% of HMOs as prototypes in the feces of breast milk-fed infants. In 2000, some scholars found that HMOs were resistant to hydrolysis by enzymes in the digestive tract of infants. This phenomenon fully demonstrates that the biological role of HMOs is not to provide material and energy to infants in the ordinary sense.

Since the 21st century, with the advancement of research methods, it has been found that the concentration of total HMOs (acidic and neutral oligosaccharides) in breast milk decreases with the prolongation of lactation period; it has also been found that HMOs are very important for the growth and development of infants, as well as current and long-term health. HMOs can facilitate the microecological balance in the intestinal tract of infants, promote the proliferation of beneficial bacteria in the intestinal tract, inhibit the growth of harmful microbes, resist pathogenic microbial infections, impede the colonization of pathogenic microbes, prevent inflammatory colorectal diseases and gastroenteritis, regulate the immune system, and promote cognitive development of infants, etc. Therefore, timely supplementation of HMOs (acidic and neutral oligosaccharides) is beneficial to the healthy growth of infants.

Among the three major classes of biological macromolecules, the synthesis of saccharides has always been a great challenge for synthetic workers, because from the perspective of the synthesis of saccharide chains in organisms, the synthesis of this type of molecules does not rely on template replication, but consists of a variety of glycosyltransferases and glycosidases are jointly regulated, which in turn determines the complexity, diversity, and micro-heterogeneity of the saccharide chain structure. Fortunately, with the rapid development of glycochemistry and enzymology, as well as the extensive work done by glycochemists and glycobiologists in the field of glycosynthesis, a series of new methods and strategies for synthesizing oligosaccharides have been reported successively. However, there are still very few technologies that can achieve industrial production.

On April 2, 2020, the European Commission issued Regulation (EU) No. 2020/484 which approved the marketing of lacto-N-tetraose (LNT) as a novel food product and included it in the list of novel food authorizations established by Commission Regulation (EU) No. 2017/2470. This product, developed by Glycoma A/S, is produced by fermentation with the genetically modified *Escherichia coli* K12 DH 1 strain and is mainly used in sterilized dairy products, fermented dairy products, cereal bars, flavored beverages, processed cereal foods, infant food, etc.

Currently, infant formulae, as a general concern by the public, uses cow's or sheep's milk as a raw material. However, these milks lack the majority of HMOs which are commonly present in human milk, and thereby cannot achieve the nutritional and immune effects of breast milk for infants and young children. Addition of HMOs with physiological functions to infant formulae, artificial milk, dairy products, baby foods, and functional health products or foods for special people will greatly improve the physical health and the quality of life of the corresponding population, which represents significant social and economic values. Therefore, there is an urgent need for industrialized technologies with stable processes to produce human milk oligosaccharides to improve market demands.

Although chemical synthesis methods can effectively obtain oligosaccharides with defined structures, and many new strategies for synthesizing oligosaccharides have been developed in recent years, the synthesis of oligosaccharides with relatively complex structures still requires a large workloads and low yields. Additionally, the control of stereoselectivity and regioselectivity in the chemical synthesis of oligosaccharides is also a perplexing problem.

Biosynthetic methods have attracted widespread attention due to their advantages such as mild reaction conditions, easy separation of products, short production cycle, high efficiency, environmental friendliness, and low cost. Compared to the lengthy multiple protection-deprotection operations required for most chemical glycosylation reactions, enzymatic synthesis exhibits good stereoselectivity and regioselectivity. Therefore, scientists have attempted to synthesize HMOs using an enzyme-catalyzed system. Nishimoto et al. (Biosci. Biotech. Bioch. 2007, 71 (8), 2101-2104.) designed a multi-enzymatic, one-pot pathway for synthesizing LNBs using sucrose as the substrate, in which sucrose was catalyzed by sucrose phosphatase (EC2.4.1.7) to obtain glucose-1-phosphate (Glc-1-p), which undergoes UDP-Gal/UDP-Glc cycle to produce galactose-1-phosphate (Gal-1-p), and then catalyzed by LNBPase (EC2.4.1.211) to produce LNBs. Therefore, when the glycosyltransferases were used in the above glycosylation reaction, it required special sugar nucleotides such as UDP-glucose or UDP-galactose are used as donors. However, to prepare UDP-glucose or UDP-galactose from glucose or galactose, glucose-1-P or galactose-1-P was generated from glucose or galactose by the catalysis of a kinase, followed by enzymatic pyrophosphorylation to obtain a diphosphate donor. The process is too complicated and the production cost is relatively high, which limits the industrial application of glycosyltransferase in synthesizing HMOs.

In Chinese invention patent 201911055516.5, lacto-N-biose phosphorylase (LnbP; EC 2.4.1.211) was used to catalyze galactose-1-p derived from galactose and GlcNAc to efficiently generate Lacto-N-biose (LNB), with the reaction time being greatly shortened. Such process of synthesizing HMOs using galactose-1-P as a donor is simple and efficient, establishes the foundation for a large-scale industrial production, and has important economic values and social benefits.

In summary, in order to achieve a large-scale industrial production and thereby provide LNT, the main ingredient of HMOs, to the market, there is an urgent need to develop an efficient, low-cost, and scalable production method that can be applied to a variety of products such as dairy products, foods, drugs, cosmetics, and feeds as soon as possible.

### Summary of the Invention

### The objective of the invention:

Provided is an enzyme which is capable of cost-effectively and efficiently synthesizing lacto-N-tetraose (LNT), as well as a preparation method thereof.

### Technical solutions:

After a number of experimental validations, the applicant has discovered a new glycoside hydrolase, which belongs to the glycoside hydrolase family GH112, and exhibits excellent properties in the synthesis of lacto-N-tetraose (LNT).

The enzyme for the synthesis of lacto-N-tetraose (LNT) belongs to the glycoside hydrolase family 112 when classified according to Carbohydrate-Active Enzymes (CAZy) database.

The enzyme of the glycoside hydrolase family 112 is preferably lacto-N-biose phosphorylase or 1,3-beta-galactosyl-N-acetylhexosamine phosphorylase, EC 2.4.1.211.

Preferably, the amino acid sequence of the enzyme of the glycoside hydrolase family 112 is shown in SEQ ID NO: 1, or the enzyme of the glycoside hydrolase family 112 has the amino acid sequence of SEQ ID NO: 1, or comprises the amino acid sequence of a lactose-N-disaccharide phosphorylase as shown in SEQ ID NO:1. Preferably, the enzyme of the glycoside hydrolase family 112 is derived from *Bacillus mycoides* (lnbp, Genebank No. QWJ02347.1) or *Bacillu cereus* (lnbp, Genebank No. WP _078422288.1).

Preferably, the amino acid sequence of the enzyme of the glycoside hydrolase family 112 is shown in SEQ ID NO:2, or the enzyme of the glycoside hydrolase family 112 has the amino acid sequence of SEQ ID NO:2, or comprises the amino acid sequence of 1,3-β-galactoside-N-acetylhexosamine phosphorylase as shown in SEQ ID NO:2. Preferably, the enzyme of the glycoside hydrolase family 112 is derived from *Bifidobacterium longum* (gnpA, Genebank No. WP_131220594.1).

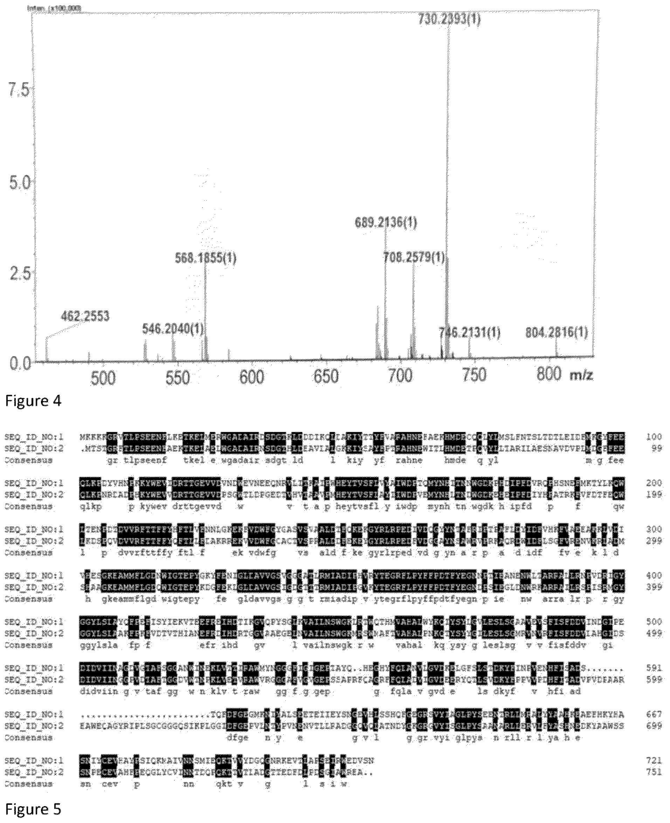

As shown in Figure 5, the amino acid sequence of SEQ ID NO: 1 has a sequence identity of 52% to that of SEQ ID NO:2. Moreover, enzymes with an amino acid sequence of SEQ ID NO:1 or SEQ ID NO:2 have the ability of catalytically synthesizing lacto-N-tetraose (LNT). It is noted that the enzyme of the glycoside hydrolase family with an amino acid sequence equivalent to any of the two amino acid sequences belongs to the scope of protection of the present patent.

The term "sequence identity" refers to a correlation between two amino acid sequences or between two nucleotide sequences, which is described by the parameter "sequence identity", also called "sequence homology".

The enzyme of the GH112 family preferably comprises an enzyme with an amino acid sequence having a sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or at least 100% to SEQ ID NO:1 or SEQ ID NO:2, and has the ability of catalytically synthesizing lacto-N-tetraose (LNT).

The enzyme of the GH112 family can be further characterized by its source, i.e., *Bacillus mycoides, Bacillus cereus,* or *Bifidobacterium longum.* "Derived from *Bacillus mycoides, Bacillus cereus,* or *Bifidobacterium longum"* means that the enzyme of the glycoside hydrolase family 112 is obtained from a microorganism (from wild strains isolated from nature, or variants after physical and chemical mutagenesis, or genetically engineered strains) classified as *Bacillus mycoides, Bacillus cereus,* or *Bifidobacterium longum,* or the enzyme of the glycoside hydrolase family 112 (GH112) is obtained through introducing the gene encoding any of the above amino acid sequences into a host cell by means of genetic engineering. Thus, recombinant polypeptides/proteins obtained by using host microorganisms transformed with genes encoding the amino acid sequences described above (or partially modified genes encoding the amino acid sequences described above), or having amino acid sequences equivalent thereto, also belong to the enzymes of the glycoside hydrolase family 112 (GH112) as described in the present invention.

In some embodiments, a preparation method for the enzyme of the glycoside hydrolase family 112 comprises: (a) generating the enzyme of the glycoside hydrolase family 112 by culturing a microorganism comprising the amino acid sequence of the enzyme of the glycoside hydrolase family 112 under conditions enabling the growth thereof.

Preferably, the method further comprises a step (b) of recovering the enzyme.

Preferably, the microorganism comprises wild bacteria or genetically engineered bacteria having an amino acid sequence shown in SEQ ID NO: 1 or SEQ ID NO:2, or having an amino acid sequence with a sequence identity of at least 70%, at least 75%, at least 80%, at least 85%, at least 90%, at least 95%, at least 97%, at least 98%, at least 99%, or at least 100% to SEQ ID NO: 1 or SEQ ID NO:2.

Preferably, the microorganism comprises wild *Bacillus mycoides,* or *Bacillus cereus,* or *Bifidobacterium longum,* or genetically engineered bacteria of the above strains.

Preferably, the host of the genetically engineered bacteria is any one selected from the group consisting of bacteria, fungi, and saccharomycetes;
preferably, the host of the genetically engineered bacteria is any one selected from the group consisting of *Escherichia coli, Vibrio nαtriegen, Bacillus subtilis, Bacillus licheniformis,* and *Bacillus αmyloliquefαcien;*
preferably, the host of the genetically engineered bacteria is any one selected from the group consisting of *Pichia pastoris, Saccharomyces cerevisiae, Kluyveromyces lactis,* and *Kluyveromyces marxianus*;
preferably, the host of the genetically engineered bacteria is any one selected from the group consisting of *Aspergillus oryzae, Aspergillus niger, Penicillium oxalicum,* and *Trichoderma reesei.* Preferably, the step (a) also comprises steps of culturing the obtained genetically engineered bacteria and inducing enzyme expression thereof, followed by cell fragmentation to obtain a crude enzyme liquid of the enzyme of the glycoside hydrolase family 112.

Preferably, the steps (b) of recovering the enzyme comprise steps of isolation and purification of the enzyme.

The steps of isolation and purification can be carried out by conventional protein purification methods, such as ion exchange chromatography, hydrophobic chromatography, gel filtration chromatography, salting out and the like, or a combination thereof, so as to obtain the purified enzyme of the glycoside hydrolase family 112. Further preferably, purification can also be carried out by nickel column affinity chromatography.

Another embodiment of the present invention relates to use of said enzymes of the glycoside hydrolase family 112 in the synthesis of lacto-N-tetraose.

With respect to the use, preferably, lacto-N-tetraose (LNT) is generated in an aqueous solution containing lactose and/or galactose and/or galactose-1-phosphate and lacto-N-triose II (LNTII). With respect to the use of the enzyme of the glycoside hydrolase family 112 in the synthesis of lacto-N-tetraose, preferably, a step of generating lacto-N-tetraose is included.

With respect to the use of the enzyme of the glycoside hydrolase family 112 in the synthesis of lacto-N-tetraose, preferably, the reaction substrate is selected from lacto-N-triose and lactose and/or galactose and/or galactose-1-phosphate.

With respect to the use of the enzyme of the glycoside hydrolase family 112 in the synthesis of lacto-N-tetraose, preferably, a crude enzyme liquid of the enzyme of the glycoside hydrolase family 112 can be directly used; more preferably, the enzyme of the glycoside hydrolase family 112 after being purified can be used as a catalyst.

With respect to the use of the enzyme of the glycoside hydrolase family 112 in the synthesis of lacto-N-tetraose, preferably, the enzyme of the glycoside hydrolase family 112 is one or more selected from the group consisting of lacto-N-biose phosphorylase as shown in SEQ ID NO: 1, and/or 1,3-beta-galactosyl-N-acetylhexosamine phosphorylase as shown in SEQ ID NO: 2.

With respect to the use of the enzyme of the glycoside hydrolase family 112 in the synthesis of lacto-N-tetraose, a suitable temperature for the reaction is in the range of 10-70°C; preferably, a suitable temperature for the reaction is in the range of 20-50°C; more preferably, a suitable temperature for the reaction is in the range of 30-50°C.

With respect to the use of the enzyme of the glycoside hydrolase family 112 in the synthesis of lacto-N-tetraose, preferably, lacto-N-tetraose is catalytically synthesized in a buffer system having a pH of 4-11; more preferably, lacto-N-tetraose is catalytically synthesized in a buffer system having a pH of 6-8.

With respect to the use of the enzyme of the glycoside hydrolase family 112 (GH112) in the synthesis of lacto-N-tetraose (LNT), the reaction system uses a buffer selected from a 3-morpholine propanesulfonic acid MOPS buffer (with a pH buffer range of 6.5-7.9) , a 4-hydroxyethyl piperazine ethanesulfonic acid HEPES buffer (with a pH buffer range of 6.8-8.2), a trihydroxymethyl amino methane hydrochloride Tris-HCl buffer (with a pH buffer range of 7.0-9.0), a phosphoric acid buffer, a PBS buffer (with a pH buffer range of 5.8-8.0), etc.

### Beneficial effects:

The present invention provides an enzyme of the glycoside hydrolase family 112 which has an ability to catalytically generate lacto-N-tetraose, as well as an expression method thereof.

The present invention provides a method for producing human milk oligosaccharides (HMOs) by using a glycoside phosphorylase of the glycoside hydrolase family 112 (GH112). Specifically, according to the method of the present invention, lactose and/or galactose and/or galactose-1-phosphate may react with lacto-N-triose II (LNTII) to generate lacto-N-tetraose (LNT), and the reaction is catalyzed by the enzyme of the glycoside hydrolase family 112 (GH112). The present invention provides a technique to improve the commercial availability of lacto-N-biose phosphorylase for transglycosylation of galactose into LNT, which is carried out under the optimal catalytic reaction conditions of the enzyme. Specifically, compared to the methods described in the prior art, the method of the present invention can improve the yield of the product and the amount of the final product.

The present invention relates to novel enzymes of the glycoside hydrolase family 112 (GH112) encoded by amino acid sequences derived from *Bacillus mycoides* or *Bacillus cereus* and *Bifidobacterium longum,* which can be efficiently expressed in microorganisms and then be applied to the synthesis of LNT. The technical solution of the present invention has positive significance for the industrial production of human milk oligosaccharides. Such method is environmentally friendly, efficient, sustainable, and conducive to the industrial large-scale production. The enzyme of the glycoside hydrolase family 112 (GH112) and the application thereof as provided by the present invention have extremely important economic and social values.

### Brief Description of the Drawings

Figure 1 illustrates the SDS-PAGE analysis profile of the supernatants in Example 1. Lanes 1-3 represent the supernatants from *Bacillus mycoides, Bacillus cereus,* and *Bifidobacterium longum,* respectively; Lane 4 is a Marker with molecular weights of 180, 130, 100, and 70.55 KDa from top to bottom.
Figure 2 illustrates the thin-layer chromatogram of the solution obtained upon the reaction of galactose-1-phosphate (Gal-1-P) with Lacto-N-triose II catalyzed by lacto-N-biose phosphorylase Bgap in Example 1. From left to right, Lanes 1-2 represent enzymatic reaction solutions; Lane 3 represents a standard of lacto-N-tetraose (LNT).
Figure 3 illustrates HPLC chromatograms of the solution obtained upon the reaction of galactose-1-phosphate (Gal-1-P) with Lacto-N-triose II catalyzed by lacto-N-biose phosphorylase Bgap in Example 1, and LNTII and LNT standards.
Figure 4 is a mass spectrogram of Peak B (LNT) from Figure 3.
Figure 5 illustrates a comparison result of amino acid sequences as shown in SEQ ID NO: 1 and SEQ ID NO: 2.

### Detailed Description of the Invention

The experimental methods used in the following examples, unless otherwise specified, are all conventional methods; all materials, reagents, etc., unless otherwise specified, can be commercially available.

The plasmids pET-32a, pEB03, pPIC9K, pklac1, and pKD1 are purchased from Shenggong Biotechnology (Shanghai) Co., Ltd.

The strains of species involved in the present invention can either be purchased commercially or can be readily obtained from the domestic and foreign culture preservation centers, such as China General Microbiological Culture Collection Center (CGMCC), China Center for Type Culture Collection (CCTCC), and Guangdong Microbiological Culture Collection Center (GDMCC), etc. The present invention will be further illustrated in detail in the following examples. It should be understood that the specific embodiments described herein are only for the purpose of explaining the present invention and are not intended to limit the present invention. Modifications or substitutions made to the details and forms of the technical solutions without deviating from the structural ideas and scope of use of the present invention will fall within the scope of protection of the present invention.

Unless otherwise defined, all technical and scientific terms used in the description have the same meanings as those commonly understood by those skilled in the art. In general, the nomenclature used in the description and experimental methods described below are well-known and commonly used in the art.

In the following Examples and Experimental Examples, HPLC analysis of products obtained by the catalyzing reaction using the enzyme of the glycoside hydrolase family 112 was performed under the following conditions: chromatographic column model: Cosmosil Sugar-D amino column (nacalai tesque, INC.); mobile phase: a 75% acetonitrile aqueous solution; column temperature: 30°C; injection volume: 10 µL; UV detector (Hitachi Chromaster), at a wavelength of 210 nm and a flow rate of 1.0 mL/min.

An ion chromatography analysis was performed under the following conditions: chromatographic column: MetroSep Carb2 (4.0 mm × 250 mm); eluent: 140 mM NaOH/20 mM NaAc; isocratic elution, at a flow rate of 0.500 mL/min; with an amperometric detector; column temperature: 40°C; injection volume: 20 µL; and running time: 50 min.

It should be noted that the terms used here are only for describing specific embodiments, and are not intended to limit exemplary embodiments according to the present invention. Furthermore, it should be also understood that when the terms "comprise(s)/comprising" and/or "include(s)/including" are used in the description, they indicate the presence of features, steps, operations, devices, components, and/or combinations thereof. It should be understood that the scope of protection of the present invention is not limited to the following specific embodiments; it should also be understood that the terms used in the examples of the present invention are intended to describe specific embodiments, rather than limiting the scope of protection of the present invention. In the following specific embodiments, if specific conditions are not specified for the experimental method, it is usually based on the conventional methods and conditions of molecular biology in the field of technology, which are fully explained in the literature. Refer to, for example, Sambrook et al., "Molecular Cloning: Experimental Manual" for techniques and conditions, or follow the manufacturer's recommended conditions.

**Example 1**. Expression of lacto-N-biose phosphorylase and 1,3-beta-galactosyl-N-acetylhexosamine phosphorylase in shake flask cultivation and purification thereof.

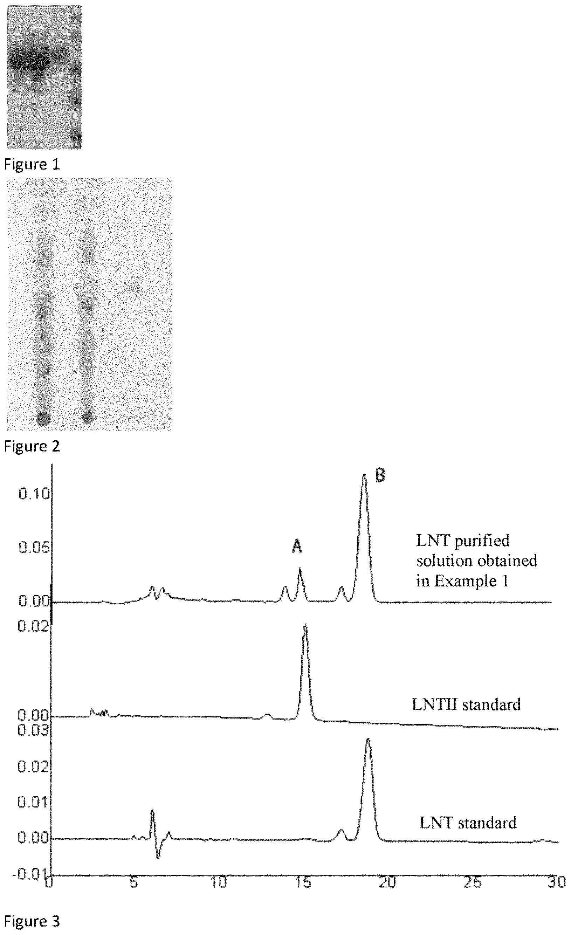

*Bacillus mycoides, Bacillus cereus* and *Bifidobacterium longum* used in this example were deposited in the ATCC strain collection center under ATCC6462, ATCC9634, and ATCC15707, respectively. *Bacillus mycoides*/*Bacillus cereus*/*Bifidobacterium longum* glycerol stocks were respectively streaked onto LB agar plates (LB medium: 1.0 % of peptone, 0.5 % of yeast extract, 1.0 % of NaCl, supplemented with 1.5 % of agar powder), and cultured overnight in an incubator at 37°C. Single colonies were picked up and put into 3 mL LB medium to culture for 6-8 h by shaking at 200 rpm at 37°C, and then 2.5 mL of the culture solution was inoculated into a 50 mL fermentation medium (1 % of tryptone, 0.5 % of yeast powder, and 1.0 % of NaCl) to culture under shaking for 72 h at 250 rpm at 34°C. Microbial cells were collected and resuspended into 50 mM PBS at pH 7.4, followed by cell fragmentation using an AH-BASIC high-pressure homogenizer until the mixture was homogeneous. The mixture was centrifuged for 30 min at 13000 rpm, the supernatant was collected and then filtered using a 0.45 µm water-based polyethersulfone membrane. The filtered supernatant was then separated by using an HiPrepTM16/60 Sephacryl TMS-300HR molecular sieve with a 50 mM PBS buffer at pH 7.4 as mobile phase at a flow rate of 0.5 mL/min. The components corresponding to the peaks at molecular weight of about 83-84 kDa were collected, and concentrated by ultrafiltration in a 10 kDa ultrafiltration tube. SDS-PAGE analysis showed that the supernatants derived from *Bacillus mycoides, Bacillus cereus,* and *Bifidobacterium longum* contained enzymes with a molecular weight of 70-100 kDa, as shown in Figure 1. It is predicted that these enzymes correspond to SEQ ID NO: 1 and SEQ ID NO: 2, and may be lacto-N-biose phosphorylase (Bgap enzyme) or 1,3-beta-galactosyl-N-acetylhexosamine phosphorylase (Blba enzyme).

The results of SDS-PAGE analysis are shown in Figure 1.

Lanes 1-3 represent supernatants from *Bacillus mycoides, Bacillus cereus,* and *Bifidobacterium longum,* respectively; Lane 4 is a Marker with molecular weights of 180, 130, 100 and 70.55 KDa from top to bottom.

As can be seen from Figure 1:
(1) The supernatants of *Bacillus mycoides* and *Bacillus cereus* contain Bgap enzyme as shown in SEQ ID NO: 1;
(2) The supernatant of *Bifidobacterium longum* contains Blba enzyme as shown in SEQ ID NO: 2. The ability of lacto-N-biose phosphorylase/1,3-beta-galactosyl-N-acetylhexosamine phosphorylase to catalyze the synthesis of LNT was determined:
20 mmol/L of galactose-1-phosphate (Gal-1-P), 10 mmol/L of lacto-N-triose LNTII, 1 mmol/L of MgCl₂, 0.2 mg of Bgap/Blba enzyme were uniformly mixed in 100 mM Tri-HCl buffer and reacted for 2 hours at 37°C at pH 7.0. Then the reaction was terminated and the reaction mixture was purified by gel filtration chromatography.

### Identification of catalytic products:

The products obtained from the catalytic reaction by Bgap/Blba enzyme were analyzed by TLC, HPLC, and LC-MS.

### (1) TLC analysis

TLC analysis was performed using a TLC plate manufactured by Merck Company (Merck Kieselgel 60 F254), with a solution of *n*-butanol: anhydrous ethanol: ultrapure water (5:3:2) as the developing solvent. After drying of the TLC plate, spots on the TLC plate were confirmed by UV light, as shown in Figure 2 for details. In Figure 2, Lanes 1 and 2 represent the purified reaction solution catalyzed by lacto-N-biose phosphorylase (Bgap) obtained in this example; Lane 3 represents LNT standard.

### (2) HPLC analysis

The HPLC analyzer and analysis conditions were shown as above.

LNT, LNnT, and LNTII standards were purchased from the agent Seebio Biotech (Shanghai) Co., Ltd., and the origin is ELICITYL, France. HPLC and/or mass spectrometry detections were performed on LNT, LNnT and LNTII standards, and the reaction solution after purification under the reaction condition of 37°C in this example, respectively.

The HPLC analysis conditions were shown previously, and the analysis results showed that:
(1) The retention time (rt) was 15.025 min for the LNTII standard, and 18.747 min for both of the LNT and LNnT standards.
(2) For the purified reaction solution catalyzed by the Bgap enzyme expressed by *Bacillus mycoides* or *Bacillus cereus,* a strong absorption peak (peak B) appeared near 18.740 min, which was consistent with the LNT standard, as shown in Figure 3; and a strong absorption peak (peak A) appeared near 15.025 min, which was consistent with the LNTII standard, and might represent incompletely reacted substrate.
   On this basis, it was concluded that the enzyme-catalytic reactions might have produced lacto-N-neotetraose (LNnT) or lacto-N-tetraose LNT.
(3) Ion chromatography was analyzed as above, and the analysis results showed that:
   The retention time was 17.09 min for the lacto-N-triose (LNTII) standard, 27.183 min for the lacto-N-tetraose (LNT) standard, and 31.086 min for the lacto-N-neotetraose (LNnT) standard.

For the purified reaction solution catalyzed by the Bgap enzyme expressed by *Bacillus mycoides* or *Bacillus cereus,* a strong absorption peak appeared near 27.183 min, which was consistent with the LNT standard, suggesting that the reactions might have produced lacto-N-tetraose (LNT); and there was no peak near 31.086 min, suggesting that none of the reactions produced lactose-N-neotetraose (LNnT).

Therefore, it is preliminarily determined that in the HPLC spectrum of the catalytic reaction solution of Bgap enzyme expressed by *Bacillus mycoides* or *Bacillus cereus,* the substance with the absorption peak at the retention time of 18.747 min (peak B) may be lacto-N-tetraose (LNT).

### (4) LC-MS analysis

LC-MS analysis was performed on the product with the absorption peak at the retention time of 18.747 min (peak B) in the HPLC spectrum. Where, the conditions for LC-MS analysis were the same as those for HPLC; H-ESI mode, molecular weight scanning range 400-900.

As shown in Figure 4, the molecular showing molecular weight of 708.2567 should be LNT+H; while the one with molecular weight of 730.2393 should be LNT + Na. Mass spectrometry results can be retrieved, and the following results were obtained: Compound CID: 440993, Chemical Formula: C₂₆H₄₅NO₂₁, Extract Mass: 707.63.

In summary, the molecular weight of the synthesized product from the enzymatic reaction with galactose-1-phosphate (Gal-1-P) and Lacto-N-triose II as substrates should be 707, and the retention time of the product was consistent with that of the LNT standard. Therefore, lacto-N-tetraose (LNT) was obtained in the catalytic reaction of lacto-N-biose phosphorylase Bgap.

Furthermore, the same conclusion was reached by analyzing the catalytic reaction solution of 1,3-beta-galactosyl-N-acetylhexosamine phosphorylase Blba obtained in this example by TLC, HPLC, and LC-MS according to the same methods mentioned above. That is to say, lacto-N-tetraose (LNT) was enzymatically synthesized by 1,3-beta-galactosyl-N-acetylhexosamine phosphorylase Blba. Therefore, it can be concluded that lacto-N-biose phosphorylase (Bgap)/1,3-beta-galactosyl-N-acetylhexosamine phosphorylase (Blba) can catalyze the synthesis of lacto-N-tetraose (LNT, MW=707.637) from galactose-1-phosphate (Gal-1-P) and lacto-N-triose II.

The concentrations of LNT in the purified reaction solutions were determined by HPLC analysis, and the results were as follows: the concentrations of LNT in the catalytic reaction solutions of Bgap enzymes (as shown in SEQ ID NO: 1) derived from *Bacillus mycoides* and *Bacillus cereus* were both 6.6 mg/ml, and the concentration of LNT in the catalytic reaction solution of Blba enzyme (as shown in SEQ ID NO: 2) derived from *Bifidobacterium longum* was 0.8 mg/mL.

**Example 2.** Protein expression of lacto-N-biose phosphorylase/1,3-beta-galactosyl-N-acetylhexosamine phosphorylase in *Escherichia coli.*

Referring to the method recited in "Guidelines for Molecular Cloning Experiments", *Bacillus mycoides* genomic DNA was used as a template, in which the gene encoding lacto-N-biose phosphorylase was temporarily named *bgap,* and the amino acid sequence was shown in SEQ ID NO: 1. The upstream primer was designed as ATCGGATCCGATTCAAAAGAAAAAAAAGGCCGTTACTTACCGAGT; and the downstream primer was designed as TGCGGCCGCAAGCTTTGCCTTCATTTGAAACATC. PCR amplification was performed to obtain a target gene fragment of 2172 bp in size. The PCR product was subjected to agarose gel electrophoresis, and the target gene *bgap* was recovered with a gel recovery kit, and then the obtained target gene *bgap* and the plasmid pET-32a were both double-digested with two restriction enzymes (*EcoR* I and *Hind* III). The digestion system was as follows: 2 µL for each of the target gene *bgap* and the plasmid pET-32a, 2 µL for each of *EcoR* I and *Hind* III, 5 µL of 10 × digestion buffer, supplemented to 50 µL by deionized water, and the digestion was performed at 37°C. The product was subjected to agarose gel electrophoresis, and then target bands on the gel were recovered with a kit. The digested target gene was ligated with the vector using DNA ligase Ligation high, and then reacted in a water bath for 30 min to obtain recombinant expression plasmid pET-32a-Bgap.

The above recombinant plasmid which was verified to be correct was transferred into *Escherichia coli* BL21 (DE3) according to the following steps: the prepared *Escherichia coli* BL21 (DE3) competent cells were placed on ice for 30 min to melt, and 100 µL of the competent cells and 10 µL of the pET-32a-Bgap recombinant plasmid (with a concentration of 50 ng/µL) were mixed uniformly and then subjected to heat shock in a water bath at 42°C for 45 s, followed by immediately cooling in an ice bath for 2 min. 1 mL of fresh LB medium (LB medium: peptone 1.0%, yeast extract 0.5%, NaCl 1.0%. For plate, add 1.5% agar powder) was added, and the cells were resuscitated and cultured at 37°C at 100 rpm for 1 h. Then 100 µL of microbial solution was coated onto a LB plate containing ampicillin (100 µg/ml), then cultured for 12 h in a thermostatic incubator at 37°C, and single colonies were picked up and recombinants were identified by PCR and double enzyme digestion to verify whether the pET-32a-Bgap recombinant plasmid was successfully constructed. Then, the positive transformants on the above plate were picked up and inoculated into LB liquid culture medium to culture for 12 h on a shaker at 200 rpm at 37°C to obtain a seed solution; the seed solution was inoculated into fresh LB culture medium at an inoculation amount of 1% (v/v) to culture with shaking at 37°C until OD₆₀₀ was 0.8. The culture was induced by addition of isopropyl β-D-thiogalactopyranoside (IPTG)(its final concentration is 0.1 mmol/L), and the induction was carried out at 16°C for 12 h at a rotation speed of 200 rpm. After induced expression, the fermentation broth was centrifuged at 5000 rpm at 4°C for 30 min to collect microbial cells. The microbial cells were resuspended into a 20 mM PBS buffer (pH7.4), and then subjected to ultrasound treatment at a frequency of plus on 5s/off 5s for 30 min to disrupt the bacteria; the disrupted mixture was centrifuged for 30 min at 13000 × g at 4°C to remove cell debris and then the supernatant was collected.

Soluble lacto-N-biose phosphorylase was purified by nickel column affinity chromatography. The process was as follows: the nickel column was naturally eluted by adding deionized water up to the top of the nickel column, and then eluted with 5-fold column volume of Binding buffer; a crude enzyme solution filtered through a 0.45 µm filter membrane was then loaded onto the column at a flow rate of 1.5 mL/min to sufficiently bound to the nickel column. After the sample run out, a 5-fold column volume of continuous gradient Washing buffer was used to remove the impurity proteins. Finally, the target protein was eluted with 5-fold column volume of Elution buffer, and the eluate was collected. Then, the expression of the target protein was analyzed by SDS-PAGE. After being induced, the SDS-PAGE results of genetically engineered bacteria showed obvious specific expression bands, and the molecular weight of the bands is basically consistent with the expected molecular weight of 102 kD, which means the protein obtained was lacto-N-biose phosphorylase Bgap as shown in SEQ ID NO: 1. Referring to the preparation method of lacto-N-biose phosphorylase Bgap shown in SEQ ID NO: 1 as above, the upstream primer was designed as ATCGGATCCGATTCAAAAAAAAAACCAGACCGGCCTTACCCT, the downstream primer was designed as TGCGGCCGCAAGCCTTCGCCACGAATGCCGCT to construct a recombinant plasmid pET-32a-Blba and then this plasmid was transformed into *E. coli* BL21 (DE3). The protein, 1,3-beta-galactosyl-*N*-acetylhexosamine phosphorylase Blba as shown in SEQ ID NO: 2 was obtained after protein expression and purification.

The ability of lacto-N-biose phosphorylase/1,3-beta-galactosyl-N-acetylhexosamine phosphorylase to catalyze the synthesis of LNT was determined:
According to the method for the synthesis of LNT catalyzed by lacto-N-biose phosphorylase/1,3-β-galactosyl-*N*-acetylhexosamine phosphorylase in Example 1, the reaction was performed for 2 h at 37°C. Upon completion of the reaction, the reaction solution was purified by a gel filtration chromatography and analyzed by HPLC. The results showed that there was a peak near 18.740 min, which was consistent with the peak time of LNT standard (18.747 min), indicating that the reaction substrates were catalyzed by lacto-N-biose phosphorylase/1,3-beta-galactosyl-*N*-acetylhexosamine phosphorylase to synthesize LNT.

The concentrations of LNT in the purified reaction solutions were analyzed by HPLC. The concentration of LNT in the catalytic reaction solution of Bgap enzyme shown in SEQ ID NO:1 was 5.3 mg/mL, and the concentration of LNT in the catalytic reaction solution of Blba enzyme shown in SEQ ID NO:2 was 0.7 mg/mL.

**Example 3.** Protein expression of lacto-N-biose phosphorylase/1,3-beta-galactosyl-N-acetylhexosamine phosphorylase in *Vibrio natriegen.*

*Vibrio natriegen* competent cells were obtained by the following steps: single colonies of *Vibrio natriegen* were picked up from a newly activated plate, inoculated into 5 mL LB liquid culture medium (tryptone 10 g/L; yeast extract 5 g/L; NaCl 10 g/L) and cultured with shaking at 37 °C for about 12 h until the logarithmic growth phase was reached. The microbial suspension was transferred to 100 mL LB liquid medium at a ratio of 1:100 to 1:200 and incubated overnight. The culture was grown at 37 °C with shaking at 200 rpm until OD₆₀₀ was 0.5. Then, the culture solution was added into two separate 250 mL centrifuge bottles respectively and cooled on ice for 15 min. Next, the culture solution was centrifuged at 4 °C at 6500 rpm for 20 min. The supernatant was discarded and the cell pellet was slightly resuspended into 5-10 mL of buffer (680 mM sucrose, 7 mM K₂PO₄, pH 7) for electroporation, blown and mixed well. The mixture was centrifuged at 4°C at 6750 rpm for 15 min. The supernatant was washed 3 times with a pipette. After the last washing, the cells were suspended in the remaining buffer. OD₆₀₀ was adjusted to 16 using the above buffer to obtain *Vibrio natriegen* competent cells. 1 µL of the pET-32a-Bgap recombinant plasmid (50 ng/µL) prepared in Example 1 and 100 µL of the competent cells were gently mixed in a 1.5 mL pre-cooled centrifuge tube. The above mixed solution was transferred to a 1-mm electroporation cuvette on a sterile operating platform, which was then placed on ice for 1 min, and quickly put into an electroporation instrument, with the parameters of 800-900 V, 25 µF, 200 ohms, and an electric shock time of 0.6 ms. After the mixture was electrically shocked, 500 µL of resuscitation culture medium (BHI culture medium with v2 salt (204 mM NaCl, 4.2 mM KCl, and 23.14 mM MgCl₂) was added and the mixture was transferred to a 15 mL culture tube to continuously incubate at 30-37 °C for 1-2 h. The resuscitation culture was coated onto a hot LB agar plate containing corresponding antibiotics and incubated at 37 °C overnight until colonies appeared. The bacterial colonies were picked up and inoculated in TBv2 culture medium (12 g/L tryptone, 24 g/L yeast extract, 0.5 % v/v glycerol, 15 g/L NaCl, 2.31 g/L KH₂PO4, 16.43 g/L K₂HPO4•3H₂O) for an extended culture on a shaker at 30°C. The positive transformants of *Vibrio natriegen* were obtained, and protein expression and purification were performed according to the method in Example 1, to obtain lacto-N-biose phosphorylase Bgap protein shown in SEQ ID NO: 1. Referring to the preparation method of lacto-N-biose phosphorylase Bgap shown in SEQ ID NO: 1 as above, the recombinant plasmid pET32a-Blba was transformed into *Vibrio natriegen* (CICC10908). After protein expression and purification, 1,3-beta-galactosyl-N-acetylhexosamine phosphorylase Blba shown in SEQ ID NO:2 can be obtained.

The ability of lacto-N-biose phosphorylase/1,3-beta-galactosyl-N-acetylhexosamine phosphorylase to catalyze the synthesis of LNT was determined:
According to the method for the synthesis of LNT catalyzed by lacto-N-biose phosphorylase/1,3-β-galactosyl-N-acetylhexosamine phosphorylase in Example 1, the reaction was performed for 2h at 37 °C. Upon completion of the reaction, the reaction solution was purified by a gel filtration chromatography and analyzed by HPLC. The results showed that there was a peak near 18.740 min, which was consistent with the peak time of LNT standard (18.747 min), indicating that LNT was synthesized under the catalysis of lacto-N-biose phosphorylase/1,3-beta-galactosyl-N-acetylhexosamine phosphorylase. The concentrations of LNT in the purified reaction solutions were analyzed by HPLC analysis. The concentration of LNT in the catalytic reaction solution of Bgap enzyme shown in SEQ ID NO:1 was 5.6 mg/mL, and the concentration of LNT in the catalytic reaction solution of Blba enzyme shown in SEQ ID NO:2 was 0.75 mg/mL.

**Example 4.** Protein expression of lacto-N-biose phosphorylase/1,3-beta-galactosyl-N-acetylhexosamine phosphorylase in *Bacillus subtilis.*

Referring to the gene sequence in Example 1, the upstream primer was designed as GATAAGCTTATTATGCCTATGAAAAAAAAAGGCGTTACTTACCGAGGAGTGAA and the downstream primer was designed as CAGGAATTCCCTTCATTGAAACATCCTCCC. PCR was performed in a 50 µL reaction system comprising: 1 µL (approximately 50 ng) of DNA template (total *Bacillus mycoides* DNA), 25 µL of 2 × Phanta Max Buffer, 1 µL of 10 pmol/ µL dNTP Mix, 2 µL for each of 10 pmol/µL forward and reverse primers, 1 µL of 1 U/µL Phanta Max Super-Fidelity DNA Polymerase, with ultrapure water supplemented to 50 µL. PCR reaction procedure was as follows: 95 °C for 3 min; 95 °C for 15 s, 58 °C for 15 s, 72 °C for 2.5 min, 30 cycles; 72 °C for 5 min; stored at 4 °C. The PCR fragment and the plasmid pEB03 were both double-digested using restriction endonucleases *EcoR* I and *Hind* III, and the digested products were ligated using a T4 ligase, and the ligation mixture was then transformed into *Escherichia coli* DH5α. Through screening and identification, the recombinant plasmid pEB03-Bgap was obtained. Then the pEB03-Bgap plasmid was transformed into *Bacillus subtilis* as follows: a full ring of *Bacillus subtilis* glycerol stock was streaked on an LB plate (LB medium: peptone 1.0 %, yeast extract 0.5 %, NaCl 1.0 %, supplemented with 1.5 % agar powder) to culture overnight in an incubator at 37 °C. Single colonies were picked into 1 mL of LB culture medium and cultured overnight at 37 °C at 200 rpm, and then 200 µL of the culture was transferred to 200 mL growth culture medium (growth culture medium: LB medium supplemented with 0.5 M sorbitol) to be cultured overnight at 37 °C at 200 rpm until OD₆₀₀ reached 0.85-0.95. Then the culture was placed in an ice bath for 10 min, centrifuged for 5 min at 4 °C at 5000 rpm, washed 4 times with pre-cooled medium (LB culture medium supplemented with 0.5 M sorbitol and 10 % glycerol), and finally resuspended in 2 mL of pre-cooled medium. The above treated microbial solution was subpackged into 80 µL for each tube, to which 1 µL (about 50 ng) of pEB03-Bgap plasmid was added. The mixture was transferred to a pre-cooled electroporation cuvette, which was then placed on ice for 1-1.5 min, and then subjected to electroporation in an electroporation instrument with the parameters set as follows: voltage 2000 V, resistance 200 Ω, followed by an ice bath for 2 min, and then 1 mL of recovery culture medium (recovery culture medium: LB culture medium supplemented with 0.5 M sorbitol and 0.38 M mannitol) was added. The mixture was incubated at 37 °C for 90 min by shaking at 200 rpm, and the microbial solution was coated onto a chloramphenicol-containing (5 µg/mL) LB plate, and then positive transformants were picked out to obtain the target *Bacillus subtilis* genetically engineered strain.

Single colonies containing positive transformants were picked up from the LB plate containing 5 µg/mL chloramphenicol and individually inoculated onto 50 mL seed culture medium (yeast extract 0.5 %, tryptone 0.5 %, glucose 1 %, K₂HPO₄ 1.8 %, chloramphenicol 5 µg/mL) and cultured with shaking at 34 °C at 210 rpm for 8 h. 2.5 mL of the culture was inoculated into 50 mL fermentation medium (tryptone 1.0 %, yeast powder 0.5 %, NaCl 1.0 %) and cultured with shaking for 48 h at 34 °C at 250 rpm. The supernatant was subjected to SDS-PAGE electrophoresis to obtain the lacto-N-biose phosphorylase Bgap protein shown in SEQ ID NO: 1. Referring to the preparation method of lacto-N-biose phosphorylase Bgap shown in SEQ ID NO: 1 as above, the upstream primer was designed as GATAAGCTGCAACATGTGCAGGCTACCACCGCGCGCTTACCT, and the downstream primer was designed as CAGGAATTCCCTTCATTCCTTCGCCACGCAATGCCGCT to construct the recombinant plasmid pEB03-Blba and then this plasmid was transformed into *Bacillus subtilis* (CICC 10732). 1,3-beta-galactosyl-N-acetylhexosamine phosphorylase shown in SEQ ID NO:2 was obtained after protein expression and purification.

The ability of lacto-N-biose phosphorylase/1,3-beta-galactosyl-N-acetylhexosamine phosphorylase to catalyze the synthesis of LNT was determined:
According to the method for the synthesis of LNT catalyzed by lacto-N-biose phosphorylase/1,3-β-galactosyl-N-acetylhexosamine phosphorylase in Example 1, the reaction was performed at 37 °C for 2 h. Upon completion of the reaction, the reaction solution was purified by a gel filtration chromatography and then measured by HPLC. The results showed that there was a peak near 18.740 min, which was consistent with the peak time of LNT standard (18.747 min), indicating that LNT was synthesized under the catalysis of lacto-N-biose phosphorylase/1,3-beta-galactosyl-N-acetylhexosamine phosphorylase.

The concentrations of LNT in the purified reaction solutions were measured by HPLC. The concentrations of LNT in the catalytic reaction solutions of Bgap enzyme shown in SEQ ID NO: 1 and of Blba enzyme shown in SEQ ID NO:2 were 6.6 mg/mL and 0.85 mg/mL, respectively. **Example 5.** Protein expression of lacto-N-biose phosphorylase/1,3-beta-galactosyl-N-acetylhexosamine phosphorylase in *Pichia pastoris.*

According to the codon-optimized gene sequence of Bgap for yeast expression as provided by Shenggong Biotechnology (Shanghai), the upstream primer was designed as GAAGCTTACGTATGATCTACTGGTAGATCAC, and the downstream primer was designed as TCAAGGCCTTAAGGCGCCTTAAGGTGTGGGTGCAAATTTC. PCR was performed in a 50 µL reaction system comprising: 1 µL of DNA template, 25 µL of 2 × Phanta Max Buffer, 1 µL of 10 pmol/µL dNTP Mix, 2 µL for each of 10 pmol/µL forward and reverse primers, 1 µL of 1 U/µL Phanta Max Super-Fidelity DNA Polymerase, with ddH₂O supplemented to 50 µL. PCR reaction procedure was as follows: 95 °C for 3 min; 95 °C for 15 s, 60 °C for 15 s, 72 °C for 1.5 min, 33 cycles; 72 °C for 5 min; stored at 4 °C. The PCR fragment and the pPIC9K plasmid were both double-digested using restriction endonucleases *Snab* 1 and *Not* I, and the digested products were ligated using a ligation high ligase, and the ligation mixture was then transformed into *Escherichia coli* DH5α. Recombinant plasmid pPIC9K-Bgap was obtained through ampicillin resistance screening.

Then, a PIC9K-Bgap expression cassette was amplified by the following steps: based on the sequence of the pPIC9K plasmid, the upstream primer was designed as GACGCAGATCGGGGAACACTGAAAAAAT; the downstream primer was designed as ACTTTGATTTCTGGATTCGGAATGGTTGAACG. PCR was performed in a 50 µL reaction system comprising: 1 µL (approximately 50 ng) of DNA template, 25 µL of 2 × Phanta Max Buffer, 1 µL of 10 pmol/µL dNTP Mix, 2 µL for each of 10 pmol/µL forward and reverse primers, 1 µL of 1 U/µL Phanta Max Super-Fidelity DNA Polymerase, with ddHzO supplemented to 50 µL. PCR reaction procedure was as follows: 95 °C for 3 min; 95 °C for 15 s, 58 °C for 15 s, 72 °C for 1.5 min, 33 cycles; 72 °C for 5 min; stored at 4 °C. The PCR products were subjected to agarose gel electrophoresis, and then the gel was cut and recovered to obtain the PIC9K-Bgap expression cassette.

The PIC9K-Bgap expression cassette was transformed into *Pichia pastoris* through the following steps:
Freshly cultured *Pichia pastoris* colonies were picked into 1 mL YPD culture medium and cultured for 12-14 h in a thermostatic oscillator at 250 rpm at 30 °C; the culture solution was transferred to 50 mL fresh YPD culture medium with an initial inoculation OD of 0.2, incubated at 30 °C by shaking at 200 rpm for 3-4 h until the OD value was 0.8-1; the yeast culture was placed on ice for 5-10 min; additionally, 25 µL of salmon sperm DNA (2 mg/mL) was placed in a PCR instrument at 95 °C for 10 min and then quickly cooled in an ice water bath, and placed on ice for later use; a solution of Bgap expression cassette to be transformed was prepared, a total of 5-10 µg was added to each tube, supplemented to 50 µL with sterilized ddH₂O. The above yeast culture on ice was centrifuged for 5 min at 4 °C at 5000 × g, the supernatant was discarded, and the cell pellet was collected and resuspended in sterilized ddH₂O. Then the cell suspension was centrifuged for 5 min at 4 °C at 5000 × g, the supernatant was discarded, and the cell pellet was collected, again; 1 mL of 0.1 M LiCl solution was added to resuspend the cell pellet and the obtained cell suspension was transferred to a new 1.5 mL centrifuge tube to centrifuge for 15 s at 4 °C at 13000 ×g, and then the supernatant was discarded. 400 µL of 0.1 M LiCl solution was added to resuspend the cell pellet and then the cell suspension was subpackaged into tubes with 50 µL per tube, the mixture was centrifuged for 15 s at 4 °C at 13000 × g, then the supernatant was discarded and the precipitate was placed on ice for later use. 240 µL of 50 % PEG3350 solution, 36 µL of 1 M LiCl solution, 25 µL of pretreated salmon sperm DNA solution, and 50 µL the solution of Bgap expression cassette to be transferred were added into each tube, and the above mixed solution was subjected to vigorous vortex and uniform mixing until the yeast cells were completely distributed (about 5 min); the thoroughly mixed system was placed in a water bath at 30 °C for 30 min, then transferred to a thermostatic water bath pot at 42 °C to heat shock for 25 min. The system was cooled to room temperature, and centrifuged for 2 min at 8000 × g. The cell pellet was collected and resuspended in 1mL YPD culture medium, and the cell suspension was subjected to shaking culture for 1-4 h at 30 °C. 25-100 µL of the culture solution was coated onto a YPD culture plate with G418 geneticin resistance; the plate was then placed in a thermostatic incubator at 30 °C to inverted culture for 2-3 days, and transformants were picked up to verify whether the expression cassette has been successfully transferred.

### Fermentation in Shaking Flask:

1) single colonies were picked up and inoculated into a 100 mL baffled flask containing 10 mL BMGY to incubate at 28-30 °C at 250-300 rpm until OD₆₀₀ reached 2-6 (approximately 16-18 hours);
2) 10 mL of the culture solution was inoculated into a 2 L shaking flask containing 1 L BMGY, and then shaken vigorously at 28-30 °C (250-300 rpm) until the logarithmic growth phase (OD₆₀₀ was 2-6) was reached; 3) the supernatant was discarded and the cells were resuspended in BMGY culture medium (100-200 mL) with a volume of 1/5-1/10 of the original volume for induction of the expression; 4) the culture solution was added to a 1 L baffled flask, which was covered with 2 layers of sterile gauze or cheesecloth, and placed in a shaker at 28-30 °C for further cultivation; 5) methanol was added to a concentration of 0.5 % every 24 hours until the optimal induction time was reached; 6) the culture solution was centrifuged for 5 min at room temperature at 1500-3000 g, and cells were collected. For intracellular expression, the supernatant was discarded, and cells were instantly processed or stored at -80 °C. For secretory expression, the supernatant was retained and placed at 4 °C for pre-cooling, and was concentrated if necessary. Next, the protein was purified according to the method in Example 1, or the supernatant was stored at -80 °C for later use. The obtained protein was lacto-N-biose phosphorylase Bgap shown in SEQ ID NO: 1. Referring to the preparation method of lacto-N-biose phosphorylase Bgap shown in SEQ ID NO: 1 as above, the upstream primer was designed as GAAGCTTACGTAACATCAACAGCGATTCACA and the downstream primer was designed as TCAAGGCCTTCGCCAGGCGAT to construct the recombinant plasmid pPIC9K-Blba, and the expression cassette PIC9K-Blba was transformed into *Pichia pastoris* (CICC1960). 1,3-beta-galactosyl-N-acetylhexosamine phosphorylase Blba shown in SEQ ID NO: 2 was obtained after expression and purification of protein.

The ability of lacto-N-biose phosphorylase/1,3-beta-galactosyl-N-acetylhexosamine phosphorylase to catalyze the synthesis of LNT was determined:
According to the method for the synthesis of LNT catalyzed by lacto-N-biose phosphorylase/1,3-β-galactosyl-N-acetylhexosamine phosphorylase in Example 1, the reaction was performed at 37 °C for 2 h. Upon completion of the reaction, the solution was purified by a gel filtration chromatography and analyzed by HPLC. The results showed that there was a peak near 18.740 min, which was consistent with the peak time of LNT standard (18.747 min), indicating that LNT was synthesized under the catalysis of lacto-N-biose phosphorylase/1,3-beta-galactosyl-N-acetylhexosamine phosphorylase. The concentrations of LNT in the purified reaction solutions were measured by HPLC analysis. The concentration of LNT in the catalytic reaction solution of Bgap enzyme shown in SEQ ID NO: 1 was 2.3 mg/mL, and the concentration of LNT in the catalytic reaction solution of Blba enzyme shown in SEQ ID NO:2 was 0.8 mg/mL.

**Example 6.** Protein expression of lacto-N-biose phosphorylase/1,3-beta-galactosyl-N-acetylhexosamine phosphorylase in *Kluyveromyces lactis.*

The recombinant plasmid pklac 1-Bgap was constructed as follows:
According to the codon-optimized gene sequence of Bgap for yeast expression as provided by a gene company, the upstream primer was designed as AGCTAGAAGAGCTAGATCTCCTAGGATGACTTCTACTGGTAGATTCACTTTGC, and the downstream primer was designed as TCAAGGCCTTAATTAAGCGGCCGCTTAGTGGTGATGGTGGTGATGCAAA. PCR was performed in a 50 µL reaction system comprising: 1 µL of DNA template (the plasmid provided by the gene company), 25 µL of 2 × Phanta Max Buffer, 1 µL of 10 pmol/µL dNTP Mix, 2 µL for each of 10 pmol/µL forward and reverse primers, 1 µL of 1 U/µL Phanta Max Super-Fidelity DNA Polymerase, with ddH₂O supplemented to 50 µL. PCR reaction procedure was as follows: 95 °C for 3 min; 95 °C for 15 s, 60 °C for 15 s, 72 °C for 1.5 min, 33 cycles; 72 °C for 5 min; stored at 4 °C. The PCR fragment and the plasmid were both double-digested using restriction endonucleases *Xmaj* I and *Not* I, and the digested products were ligated using a ligation high ligase, and the ligation mixture was then transformed into *Escherichia coli* DH5α. Recombinant plasmid pklac1-Bgap was obtained by screening and identification.

Then, a klac1-Bgap expression cassette was amplified by the following steps: based on the sequence of the pklac1 plasmid, the upstream primer was designed as CCGCGGGGATCGACTCATAAAATAGTAACCTTCT; the downstream primer was designed as GCCGCGGAAATTTAGGAATTTTAAACTTGGGCTTG. PCR was performed in a 50 µL reaction system comprising: 1 µL (approximately 50 ng) of DNA template (pklac1-Bgap plasmid), 25 µL of 2 × Phanta Max Buffer, 1 µL of 10 pmol/µL dNTP Mix, 2 µL for each of 10 pmol/µL forward and reverse primers, 1µL of 1 U/µL Phanta Max Super-Fidelity DNA Polymerase, with ddH₂O supplemented to 50 µL. PCR reaction procedure was as follows: 95 °C for 3 min; 95 °C for 15 s, 60 °C for 15 s, 72 °C for 5 min, 33 cycles; 72 °C for 5 min; stored at 4 °C. The PCR products were subjected to agarose gel electrophoresis, and then the gel was cut and recovered to obtain the klac1-Bgap expression cassette.

Afterwards, the klac1-B gap expression cassette was transformed into *Kluyveromyces lactis.* The specific operations were as follows: freshly cultured *Kluyveromyces lactis* colonies were picked into 1 mL of YPD culture medium and cultured for 12-14 h in a thermostatic oscillator at 250 rpm at 30 °C; the culture solution was transferred to 50 mL fresh YPD culture medium with an initial inoculation OD of 0.2, incubated at 30 °C at 200 rpm for 3-4 h until the OD value was 0.8-1; the yeast culture was placed on ice for 5-10 min; at the same time, 25 µL of salmon sperm DNA (2 mg/mL) was placed on a PCR instrument at 95 °C for 10 min and then quickly cooled in an ice water bath, and placed on ice for later use; a total of 5-10 µg of the expression cassette solution to be transformed was added to each tube, supplemented to 50 µL with sterilized ddH₂O. The above yeast culture on ice was centrifuged for 5 min at 4 °C at 5000 × g, the supernatant was discarded, and the cell pellet was collected and resuspended in sterilized ddHzO. Then the cell suspension was centrifuged for 5 min at 4 °C at 5000 × g, the supernatant was discarded, and the cell pellet was collected, again; 1 mL of 0.1 M LiCl solution was added to resuspend the cell pellet and the obtained cell suspension was transferred to a new 1.5 mL centrifuge tube to centrifuge for 15 s at 4 °C at 13000 ×g, and then the supernatant was discarded. 400 µL of 0.1 M LiCl solution was added to resuspend the cell pellet and then the cell suspension was subpackaged into tubes with 50 µL per tube, the mixture was centrifuged for 15 s at 4 °C at 13000 × g, then the supernatant was discarded and the precipitate was placed on ice for later use. 240 µL of 50 % PEG3350 solution, 36 µL of 1 M LiCl solution, 25 µL of pretreated salmon sperm DNA solution, and 50 µL the solution of expression cassette to be transferred were added into each tube, and the above mixed solution was subjected to vigorous vortex and uniform mixing until the yeast cells were completely distributed (about 5 min); the thoroughly mixed system was placed in a water bath at 30 °C for 30 min, then transferred to a thermostatic water bath pot at 42 °C to heat shock for 25 min. The system was cooled to room temperature, centrifuged for 2 min at 8000 × g. The cell pellet was collected and resuspended in 1 mL YPD culture medium, and the cell suspension was subjected to shaking culture for 1-4 h at 30 °C. 25-100 µL of the culture solution was coated onto a culture plate containing G418 resistance; the plate was then placed in a thermostatic incubator at 30 °C to inverted culture for 2-3 days, and transformants were picked up to verify whether the expression cassette has been successfully transferred.

Fermentation in shaking flask was performed as follows: 1) preparation of primary strains: single colonies of the above genetically engineered yeast strains were cultured overnight in 10 mL of YGD liquid culture medium at 30 °C at 200 rpm, so as to obtain the primary strains; 2) preparation of secondary strains: the primary strains were inoculated into 1 L of YGD liquid culture medium to culture at 30 °C at 200 rpm until OD₆₀₀ was around 7; 3) the YGD culture solution was centrifuged to obtain a crude solution of the target enzyme, which was then purified according to the method in Example 1 to obtain the protein lacto-N-biose phosphorylase Bgap shown in SEQ ID NO: 1. Referring to the preparation method of lacto-N-biose phosphorylase Bgap shown in SEQ ID NO: 1 as above, the upstream primer was designed as GAAGCTTACGTAACATCAACAGCGATTCACA and the downstream primer was designed as TCAAGGCCCTTAGGTTCGCCAGGCGCGCGGAT, a recombinant plasmid pklac1-Blba was constructed, the expression cassette was transformed into *Kluyveromyces lactis* (ATCC8585). 1,3-beta-galactosyl-N-acetylhexosamine phosphorylase Blba shown in SEQ ID NO: 2 was obtained after expression and purification of protein.

The ability of lacto-N-biose phosphorylase/1,3-beta-galactosyl-N-acetylhexosamine phosphorylase to catalyze the synthesis of LNT was determined:
According to the method for the synthesis of LNT catalyzed by lacto-N-biose phosphorylase/1,3-β-galactosyl-N-acetylhexosamine phosphorylase in Example 1, the reaction was performed at 37 °C for 2 h. Upon completion of the reaction, the reaction solution was purified by a gel filtration chromatography and analyzed by HPLC. The results showed that there was a peak near 18.740 min, which was consistent with the retention time of LNT standard (18.747 min), indicating that LNT was synthesized under the catalysis of lacto-N-biose phosphorylase/1,3-beta-galactosyl-N-acetylhexosamine phosphorylase. The concentrations of LNT in the purified reaction solutions were measured by HPLC, and the results were as follows: the concentration of LNT in the catalytic reaction solution of Bgap enzyme shown in SEQ ID NO:1 was 2.1 mg/mL, and the concentration of LNT in the catalytic reaction solution of Blba enzyme shown in SEQ ID NO:2 was 0.58 mg/mL.

**Example 7.** Protein expression of lacto-N-biose phosphorylase/1,3-beta-galactosyl-N-acetylhexosamine phosphorylase in *Kluyveromyces marxianus.*

Firstly, a Bgap expression cassette was constructed by the following steps: 1) Amplification of the Bgap gene fragment. According to the codon-optimized gene sequence of Bgap for yeast expression as provided by a gene company, the upstream primer was designed as CAATTACAAGAGAACTTCTACTGGTAGATTCACTTTGCCATCTG, and the downstream primer was designed as ATGGTGGTGATGCAAATTTCTCCAAGCAATAGCAGATTGATC. PCR was performed in a 50 µL reaction system comprising: 1 µL of DNA template (the plasmid provided by the gene company), 25 µL of 2 × Phanta Max Buffer, 1 µL of 10 pmol/µL dNTP Mix, 2 µL for each of 10 pmol/µL forward and reverse primers, 1 µL of 1 U/µL Phanta Max Super-Fidelity DNA Polymerase, with ddH₂O supplemented to 50 µL. PCR reaction procedure was as follows: 95 °C for 3 min; 95 °C for 15 s, 60 °C for 15 s, 72 °C for 1.5 min, 33 cycles; 72 °C for 5 min; stored at 4 °C. 2) Amplification of inulinase promoter fragment and signal peptide fragment. The genome of *Kluyveromyces marxianus* was extracted, the upstream primer was designed as CAATTACAAGAGAACTTCTACTGGTAGATTCACTTTGCCATCTG, and the downstream primer was designed as CCTCCATGTCCCGGTAGAGGTGTGGTCAAT. PCR was performed in a 50 µL reaction system comprising: 1 µL of DNA template (the plasmid provided by the gene company), 25 µL of 2 × Phanta Max Buffer, 1 µL of 10 pmol/µL dNTP Mix, 2 µL for each of 10 pmol/µL forward and reverse primers, 1 µL of 1 U/µL Phanta Max Super-Fidelity DNA Polymerase, with ddH₂O supplemented to 50 µL. PCR reaction procedure was as follows: 95 °C for 3 min; 95 °C for 15 s, 60 °C for 15 s, 72 °C for 1 min, 33 cycles; 72 °C for 5 min; stored at 4 °C. 3) Amplification of terminator fragment. The genome of *Kluyveromyces marxianus* was extracted, the upstream primer was designed as CAATTACAAGAGAACTTCTACTGGTAGATTCACTTTGCCATCTG, and the downstream primer was designed as CCTCCATGTCCCGGTAGAGGTGTGGTCAAT. PCR was performed in a 50 µL reaction system comprising: 1 µL of DNA template, 25 µL of 2 × Phanta Max Buffer, 1 µL of 10 pmol/µL dNTP Mix, 2 µL for each of 10 pmol/µL forward and reverse primers, 1 µL of 1 U/µL Phanta Max Super-Fidelity DNA Polymerase, with ddH₂O supplemented to 50 µL. PCR reaction procedure was as follows: 95 °C for 3 min; 95 °C for 15 s, 60 °C for 15 s, 72 °C for 1 min, 33 cycles; 72 °C for 5 min; stored at 4 °C. 4) Amplification of G418 resistant gene. The upstream primer was designed as CCTCTACCGGGACATGGAGGCCCAGAATAC, and the downstream primer was designed as AAGCAGATCAGACAGTATAGCGACCAGCATTC. PCR was performed in a 50 µL reaction system comprising: 1 µL of DNA template, 25 µL of 2 × Phanta Max Buffer, 1 µL of 10 pmol/µL dNTP Mix, 2 µL for each of 10 pmol/µL forward and reverse primers, 1 µL of 1 U/µL Phanta Max Super-Fidelity DNA Polymerase, with ddH₂O supplemented to 50 µL. PCR reaction procedure was as follows: 95 °C for 3 min; 95 °C for 15 s, 60 °C for 15 s, 72 °C for 1.5 min, 33 cycles; 72 °C for 5 min; stored at 4 °C. 5) Amplification of inulinase terminator. The upstream primer was designed as TCGCTATACTGTCTGATCTGCTTACTTTACTAACGAC, and the downstream primer was designed as TCGTGGCAGCAATTCATGATCGTAACGTGGTA. PCR was performed in a 50 µL reaction system comprising: 1 µL of DNA template, 25 µL of 2 × Phanta Max Buffer, 1 µL of 10 pmol/µL dNTP Mix, 2 µL for each of 10 pmol/µL forward and reverse primers, 1 µL of 1 U/µL Phanta Max Super-Fidelity DNA Polymerase, with ddH₂O supplemented to 50 µL. PCR reaction procedure was as follows: 95 °C for 3 min; 95 °C for 15 s, 60 °C for 15 s, 72 °C for 1.5 min, 33 cycles; 72 °C for 1 min; stored at 4 °C. 6) Ligation of PCR fragments. The G418 gene and the inulinase terminator gene were ligated by the abclonal ligase. The inulinase gene promoter, the signal peptide, the Bgap gene, and the terminator gene were ligated through fusion PCR. The above two fragments were further ligated through fusion PCR to obtain a Bgap expression cassette.

Then, the Bgap expression cassette was transformed into *Kluyveromyces marxianus* by the following steps: freshly cultured *Kluyveromyces marxianus* colonies were picked into 1 mL YPD culture medium and cultured for 12-14 h in a thermostatic oscillator at 250 rpm at 30 °C; the culture solution was transferred to 50 mL fresh YPD culture medium with an initial inoculation OD of 0.2, incubated at 30 °C at 200 rpm for 3-4 h until the OD value was 0.8-1; the yeast culture was placed on ice for 5-10 min; at the same time, 25 µL of salmon sperm DNA (2 mg/mL) was placed in a PCR instrument at 95 °C for 10 min and then quickly cooled in an ice water bath, and placed on ice for later use; a total of 5-10 µg of the expression cassette solution to be transformed was added to each tube, supplemented to 50 µL with sterilized ddH₂O. The yeast culture on ice was centrifuged for 5 min at 4 °C at 5000 × g, the supernatant was discarded, and the cell pellet was collected and resuspended in sterilized ddHzO. Then the cell suspension was centrifuged for 5 min at 4 °C at 5000 × g, the supernatant was discarded, and the cell pellet was collected, again; 1 mL of 0.1 M LiCl solution was added to resuspend the cell pellet and the obtained cell suspension was transferred to a new 1.5 mL centrifuge tube to centrifuge for 15 s at 4 °C at 13000 × g, and then the supernatant was discarded. 400 µL of 0.1 M LiCl solution was added to resuspend the cell pellet and then the cell suspension was subpackaged into tubes with 50 µL per tube, the mixture was centrifuged for 15 s at 4 °C at 13000 × g, then the supernatant was discarded and the precipitate was placed on ice for later use; 240 µL of 50 % PEG3350 solution, 36 µL of 1 M LiCl solution, 25 µL of pretreated salmon sperm DNA solution, and 50µL of the solution of expression cassette to be transferred were added into each tube, and the above mixed solution was subjected to vigorous vortex and uniform mixing until the yeast cells were completely distributed (about 5 min); the thoroughly mixed system was placed in a water bath at 30 °C for 30 min, then transferred to a thermostatic water bath pot at 42 °C to heat shock for 25 min. The system was cooled to room temperature, centrifuged for 2 min at 8000 × g. The cell pellet was collected and resuspended with 1 mL YPD culture medium, and the cell suspension was subjected to shaking culture for 1-4 h at 30 °C. 25-100 µL of the culture solution was coated onto a YPD culture plate with G418 resistance; the plate was then placed in a thermostatic incubator at 30 °Cto inverted culture for 2-3 days, and transformants were picked up to verify whether the expression cassette has been successfully transferred.

Finally, positive transformants were selected for use in the production of lacto-N-biose phosphorylase by the operation steps: 1) preparation of primary strains: single colonies of the above genetically engineered yeast strains were cultured overnight in 10 mL of YGD liquid culture medium with G418 at 30 °C at 200 rpm, so as to obtain the primary strains; 2) preparation of secondary strains: the primary strains were inoculated into 1 L of YGD liquid culture medium with G418, 5 g of inulin was added therein as an inducer, and the mixture was cultured at 30 °C at 200 rpm until OD₆₀₀ was around 7; 3) the YGD culture solution was centrifuged to obtain a crude solution of the target enzyme, which was then purified according to the method in Example 1 to obtain the protein lacto-N-biose phosphorylase Bgap as shown in SEQ ID NO: 1. Referring to the preparation method of lacto-N-biose phosphorylase Bgap shown in SEQ ID NO: 1 as above, the upstream primer was designed as CAATTACAAGAGAACTTCTACATGACATCAACAGGCCGATTCACA and the downstream primer was designed as ATGGTGGTGATGCAAATTTTTAGGCTTCGCGCCAGGCGAT, a Blba expression cassette was constructed and transformed into *Kluyveromyces marxianus* (ATCC46537).1,3-beta-galactosyl-N-acetylhexosamine phosphorylase Blba shown in SEQ ID NO: 2 was obtained after expression and purification of protein.

The ability of lacto-N-biose phosphorylase/1,3-beta-galactosyl-N-acetylhexosamine phosphorylase to catalyze the synthesis of LNT was determined:
According to the method for the synthesis of LNT catalyzed by lacto-N-biose phosphorylase/1,3-β-galactosyl-N-acetylhexosamine phosphorylase in Example 1, the reaction was performed at 37 °C for 2 h. Upon completion of the reaction, the reaction solution was purified by a gel filtration chromatography and analyzed by HPLC. The results showed that there was a peak near 18.740 min, which was consistent with the peak time of LNT standard (18.747 min), indicating that LNT was synthesized under the catalysis of lacto-N-biose phosphorylase/1,3-beta-galactosyl-N-acetylhexosamine phosphorylase. The concentrations of LNT in the purified reaction solutions were measured by HPLC analysis. The concentration of LNT in the catalytic reaction solution of Bgap enzyme shown in SEQ ID NO:1 was 2.1 mg/mL, and the concentration of LNT in the catalytic reaction solution of Blba enzyme shown in SEQ ID NO:2 was 0.64 mg/mL.

**Example 8.** Protein expression of lacto-N-biose phosphorylase/1,3-beta-galactosyl-N-acetylhexosamine phosphorylase in *Aspergillus oryzae.*

In this experiment, genomic DNA was extracted from *Aspergillus oryzae.* The *Aspergillus oryzae* genomic DNA was used as a template to amplify *Aspergillus oryzae* amylase promoter and glycosidase terminator. The promoter, terminator, and expression vector skeleton pCAMBIA1304 were digested and ligated so that the promoter PamyB and the terminator TagdA were ligated to the corresponding digestion sites to construct an *Aspergillus oryzae* reading frame to obtain the plasmid pCAMBIA01. *Bgap* gene and the plasmid pCAMBIA01 were separately subjected to a dual digestion using *Sal* I and *Spe* I, and the two digested fragments were ligated together. The ligated product was transformed into *Escherichia coli* competent cell DH5α, positive clones were screened and subjected to expansion culture. The plasmid pCAMBIA02 was extracted and the recombinant plasmid was sequenced to verify the target gene. The sequencing results showed that the target gene was correct.

Preparation of *Agrobacterium tumefaciens* EHA105 competent cells: 1) *Agrobαcterium tumefaciens* EHA105 activated on a solid YEB plate were inoculated into 20 mL YEB liquid culture medium containing the antibiotics rifampicin to culture with shaking at 180 rpm at 28 °C for 24 h. 2) *Agrobacterium tumefaciens* were inoculated into 50 mL of YEB liquid culture medium added with rifampicin to culture with shaking at 180 rpm at 28 °C until OD₆₀₀ was 0.8. 3) 50 mL of the culture solution was placed on ice for 30 min, centrifuged for 10 min at 5000 rpm at 4 °C, and the supernatant was discarded. 4) 10 mL of 0.1 mol/L pre-cooled CaCl₂ solution was added to resuspend the cell pellet. The cell suspension was placed on ice for 30 min and then centrifuged for 10 min at 5000 rpm at 4 °C. The supernatant was discarded. 5) 2 mL of 0.1 mol/L pre-cooled CaCl₂ solution and 2 mL of glycerol were added to resuspend EHA105 cells, and the cell suspension was subpackaged into tubes with 100 µL per tube, which were then stored in an ultra-low temperature refrigerator at -70 °C for later use.

The plasmid pCAMBIA02 was transformed into EHA105 competent cells by using a freeze-thaw method: 1) *Agrobacterium tumefaciens* EHA105 competent cells were molten on ice and 10 µL of the recombinant plasmid pCAMBIA02 was added, the materials were mixed well and placed on ice for 30 min. 2) The mixed solution was quickly placed in liquid nitrogen for 5 min of cold stimulation, then immediately placed in a thermostatic water bath pot at 28 °C and incubated for 5 min. 3) The mixed solution was placed on ice for 5 min. 4) 400 µL of YEB liquid culture medium without rifampicin was added into the mixed solution to culture with shaking at 180 rpm for 2 h at 28 °C. 5) 200 mL of the culture solution was coated onto a solid YEB culture plate containing rifampicin and kanamycin to inverted culture for 48 h at 28 °C and the formation of colonies was observed. 6) Single colonies were picked into 20 mL of YEB liquid culture medium containing rifampicin and kanamycin to culture with shaking at 180 rpm for 48 h at 28 °C. The colonies were identified by PCR so as to screen positive clones. The plasmid DNA was extracted from the positive clones for PCR detection of the plasmid.

*Agrobacterium tumefaciens* mediated the transformation of expression vectors into *Aspergillus oryzae:* 1) solutions of *Agrobacterium* EHA105 containing different expression vectors were separately transferred to LB liquid culture medium added with rifampicin and kanamycin, and cultured at 180 rpm for 24 h at 28 °C. 2) 2 mL of the culture solution was taken and added to a mixed culture medium consisting of 10 mL of LB liquid culture medium and 10 mL of basic culture medium to culture with shaking at 180 rpm for 48 h at 28 °C. 3) 2 mL of the culture solution was added into 20 mL of induction culture medium and cultured with shaking at 180 rpm at 28 °C until OD₆₀₀was 0.8, atwhichthe culture solution can be incubated with *Aspergillus oryzae.* 4) Preparation of *Aspergillus oryzae* conidia suspension. 5) 100 µL of the conidia suspension and 100 µL of EHA105 solution were evenly mixed and then the mixture was coated onto solid induction culture medium lined with a microporous filter membrane, in which the induction culture medium was supplemented with acetosyringone, followed by incubation at 28 °C for a total of 3 days. 6) The microporous filter membrane was removed and reversely paved onto Czapek-Dox culture medium containing hygromycin B and cephalosporin for antibiotic screening culture at 30 °C for 3 days. 7) The microporous filter membrane was removed and the culture was continued until resistant colonies grew up. The screened resistant colonies were transferred to another selective culture medium for further screening culture. The screened resistant transformants were verified by genomic PCR.

Expression of Bgap protein: after the constructed *Aspergillus oryzae* were activated, they were cultured at a constant temperature of 30 °C for 3 days, and the spores were washed away with sterile water to prepare a fungus suspension for inoculation. The different strains were inoculated into solid fermentation culture medium at 2 %. Further, the microbial solution was cultured in a culture medium for 6 days at 30 °C at 200 rpm to induce the protein expression and then centrifuged at 12000 rpm. The supernatant was collected and concentrated using a 3 kDa ultrafiltration membrane, followed by purification with the nickel column affinity tomography described in Example 1, to obtain the protein lacto-N-biose phosphorylase Bgap shown in SEQ ID NO:1. Referring to the preparation method of lacto-N-biose phosphorylase Bgap shown in SEQ ID NO: 1 as above, a recombinant plasmid pCAMBIA03 containing Blba expression cassette was constructed and transformed into *Aspergillus oryzae* (CICC2012). The protein, 1,3-beta-galactosyl-N-acetylhexosamine phosphorylase Blba shown in SEQ ID NO:2 was obtained after expression and purification of protein.

The ability of lacto-N-biose phosphorylase/1,3-beta-galactosyl-N-acetylhexosamine phosphorylase to catalyze the synthesis of LNT was determined:
According to the method for the synthesis of LNT catalyzed by lacto-N-biose phosphorylase/1,3-β-galactosyl-N-acetylhexosamine phosphorylase in Example 1, the reaction was performed at 37 °C for 2 h. Upon completion of the reaction, the reaction solution was purified by a gel fitration chromatography and analyzed by HPLC. The detection results showed that there was a peak near 18.740 min, which was consistent with the peak time of LNT standard (18.747 min), indicating that LNT was synthesized under the catalysis of lacto-N-biose phosphorylase/1,3-beta-galactosyl-N-acetylhexosamine phosphorylase. The concentrations of LNT in the purified reaction solutions were measured by HPLC analysis. The concentrations of LNT in the catalytic reaction solutions of Bgap enzyme shown in SEQ ID NO:1 and Blba enzyme shown in SEQ ID NO:2 were 1.8 mg/mL and 0.42 mg/mL, resprectively.

Example 9. The effect of temperature on the synthesis of LNT catalyzed by lacto-N-biose phosphorylase/1,3-beta-galactosyl-N-acetylhexosamine phosphorylase (Bgap/Blba) obtained in Example 2.

Referring to the method for the synthesis of LNT catalyzed by the lacto-N-biose phosphorylase/1,3-beta-galactosyl-N-acetylhexosamine phosphorylase in Example 1, the reaction was performed for 2 h under the individual reaction temperatures recited in Table 1 by using the purified lacto-N-biose phosphorylase/1,3-beta-galactosyl-N-acetylhexosamine phosphorylase (Bgap/Blba) obtained in Example 2. After the reaction was terminated, the reaction solution was purified using a gel filtration chromatography.

The concentration of LNT in the purified reaction solution was analyzed by HPLC and the results were shown in Table 1.

**Table 1. The effect of temperature on the synthesis of LNT catalyzed by lacto-N-biose phosphorylase/1,3-beta-galactosyl-N-acetylhexosamine phosphorylase (Bgap/Blba) obtained in Example 2**

| Temperature/°C | LNT (mg/mL) | |
|---|---|---|
| | Bgap | Blba |
| 10 | 0.6 | 0.13 |
| 20 | 1.8 | 0.32 |
| 30 | 5.0 | 0.58 |
| 40 | 5.4 | 0.81 |
| 50 | 4.1 | 0.42 |
| 60 | 0.5 | 0.32 |
| 70 | 0.1 | 0.26 |

As can be seen from Table 1, both lacto-N-biose phosphorylase and 1,3-beta-galactosyl-N-acetylhexosamine phosphorylase (Bgap/Blba) obtained in Example 2 have the ability to catalyze the synthesis of LNT at a temperature in the range of 10-70 °C, preferably in the range of 20-50 °C, and more preferably in the range of 30-50 °C.

**Example 10.** The effect of pH on the synthesis of LNT catalyzed by lacto-N-biose phosphorylase/1,3-beta-galactosyl-N-acetylhexosamine phosphorylase (Bgap/Blba) obtained in Example 2.

Referring to the method for the synthesis of LNT catalyzed by the lacto-N-biose phosphorylase/1,3-beta-galactosyl-N-acetylhexosamine phosphorylase in Example 1, the reaction was performed for 2 h at the individual pH values recited in Table 2 using the purified lacto-N-biose phosphorylase/1,3-beta-galactosyl-N-acetylhexosamine phosphorylase (Bgap/Blba) obtained in Example 2. After the reaction was terminated, the reaction solution was purified using a gel filtration chromatography. The results were shown in Table 2.

**Table 2. The effect of pH on the synthesis of LNT catalyzed by lacto-N-biose phosphorylase/1,3-beta-galactosyl-N-acetylhexosamine phosphorylase (Bgap/Blba) obtained in Example 2**

| pH | LNT (mg/mL) | |
|---|---|---|
| | Bgap | Blba |
| 5 | 1.2 | 0.13 |
| 6 | 4.5 | 0.42 |
| 7 | 5.4 | 0.75 |
| 8 | 5.4 | 0.53 |
| 9 | 1.0 | 0.21 |
| 10 | 0.6 | 0.12 |
| 11 | 0.1 | 0.05 |

As can be seen from Table 2, both lacto-N-biose phosphorylase and 1,3-beta-galactosyl-N-acetylhexosamine phosphorylase (Bgap/Blba) obtained in Example 2 have the ability to catalyze the synthesis of LNT at pH 5-11, and preferably at pH 6-8.

**Example 11.** The effect of reaction time on the synthesis of LNT catalyzed by lacto-N-biose phosphorylase/1,3-beta-galactosyl-N-acetylhexosamine phosphorylase (Bgap/Blba) obtained in Example 2.

Referring to the synthesis of LNT catalyzed by the lacto-N-biose phosphorylase/1,3-beta-galactosyl-N-acetylhexosamine phosphorylase in Example 1, the reaction was performed at 37 °C for different reaction times as recited in Table 3 using the purified lacto-N-biose phosphorylase/1,3-beta-galactosyl-N-acetylhexosamine phosphorylase (Bgap/Blba) obtained in Example 2. After the reaction was terminated, the reaction solution was purified using a gel filtration chromatography. The results were shown in Table 3.

**Table 3. The effect of reaction time on the synthesis of LNT catalyzed by lacto-N-biose phosphorylase/1,3-beta-galactosyl-N-acetylhexosamine phosphorylase (Bgap/Blba) obtained in Example 2**

| Reaction time/h | LNT (mg/mL) | |
|---|---|---|
| | Bgap | Blba |
| 2 | 5.4 | 0.71 |
| 4 | 7.7 | 1.35 |
| 12 | 10.5 | 1.50 |
| 17 | 9.4 | 1.40 |
| 20 | 9.9 | 1.41 |
| 24 | 9.5 | 1.30 |

As can be seen from Table 3, a certain amount of LNT was generated at a reaction time of 2 hours when using lacto-N-biose phosphorylase/1,3-beta-galactosyl-N-acetylhexosamine phosphorylase (Bgap/Blba) obtained in Example 2 to catalyze the synthesis of LNT, and the concentration of LNT in the reaction solution increased rapidly at the reaction time of 4-12 hours, and after that, increased slowly.

**Example 12.** The effect of enzyme amount on the synthesis of LNT catalyzed by lacto-N-biose phosphorylase/1,3-beta-galactosyl-N-acetylhexosamine phosphorylase (Bgap/Blba) obtained in Example 2.

Referring to the synthesis of LNT catalyzed by the lacto-N-biose phosphorylase/1,3-beta-galactosyl-N-acetylhexosamine phosphorylase in Example 1, the reaction was performed at 37 °C for 2 h by using the purified lacto-N-biose phosphorylase/1,3-beta-galactosyl-N-acetylhexosamine phosphorylase (Bgap/Blba) obtained in Example 2 at different usage amounts as recited in Table 4. After the reaction was terminated, the reaction solution was purified using a gel filtration chromatography. The concentration of LNT was measured by HPLC. The results were shown in Table 4.

**Table 4. The effect of enzyme amount on the synthesis of LNT catalyzed by lacto-N-biose phosphorylase/1,3-beta-galactosyl-N-acetylhexosamine phosphorylase (Bgap/Blba) obtained in Example 2**

| Enzyme addition amount/(mg/mL) | LNT/(mg/mL) | |
|---|---|---|
| | Bgap | Blba |
| 0.1 | 3.6 | 0.34 |
| 0.2 | 5.4 | 0.71 |
| 0.5 | 7.0 | 1.08 |
| 0.6 | 7.4 | 1.35 |
| 1.0 | 7.3 | 1.36 |

As can be seen from Table 4, a certain amount of LNT was generated when an enzyme addition amount was 0.1 mg/mL via lacto-N-biose Phosphorylase/1,3-beta-galactosyl-N-acetylhexosamine phosphorylase (Bgap/Blba) obtained in Example 2 catalyzing the synthesis of LNT at 37 °C for 2 hours; thereafter, the amount of LNT increased with the enzyme addition amount, and the optimum was achieved at 0.2-0.6 mg/mL of the enzyme addition amount; afterward, the effect of the enzyme addition amount on LNT production was not significant.

**Example 13.** Synthesis of LNT with lactose as a substrate under the catalysis of lacto-N-biose phosphorylase Bgap obtained in Example 2.

Lactase was purchased from Qingdao Vland Biotech Co.,Ltd. Referring to the method provided by the article (Immobilization of β-Galactosidase and Its Use in Preparing Low Lactose Milk, Food Industry, 2018, 39 (9): 105-110), 20 mmol/L lactose was dissolved in 100 mmol/L of Tris-HCl buffer (pH 6.5), to which lactase was added at a ratio of 120 U of lactase per gram of lactose and mixed well. The reaction was carried out at 37 °C for 5 hours.

Then, referring to the methods provided by Chinese invention patent 201911055516.5 and the article (LNB Synthesis via a Modular Enzymatic Cascade with ATP Regeneration. iScience 24 (3): 102236), pH was changed into 7.0 and then 20 mmol/L ATP and 20 U/ml galactokinase (Galk; EC2.7.1.6) were dissolved in the buffer solution and the mixture was reacted at 30 °C for 60 min. Continuously, 10 mmol/L Lacto-N-triose (LNTII), 10 mmol/L MgCl₂ and 0.2 mg Bgap enzyme were added to the solution and the whole mixture was stirred at 37°C for 12 hours. Finally, about 1.8 mmol/L of Lacto-N-tetraose (LNT) generated in this reaction was measured by HPLC analysis.

**Example 14.** Synthesis of LNT with galactose as a substrate under the catalysis of lacto-N-biose phosphorylase Bgap obtained in Example 1.
1) Referring to the methods provided by Chinese invention patent 201911055516.5 and the article (Lacto-N-biose Synthesis via a Modular Enzymatic Cascade with ATP Regeneration. iScience 24 (3): 102236), 20 mmol/L galactose and 20 mmol/mL ATP were dissolved in 100 mmol/L Tris-HCl buffer, 20 U/mL galactokinase (Galk; EC2.7.1.6) was added to the solution. The mixture was stirred at 30 °C for 60 min. Then, 10 mmol/L lacto-N-triose II, 10 mmol/L MgCh and 0.2 mg Bgap enzyme were added to the solution and the whole mixture was stirred at 37°C for 12 hours. Finally, about 1.6 mmol/L of Lacto-N-tetraose (LNT) generated in this reaction was measured by HPLC analysis.
2) Referring to the methods provided by Chinese invention patent 201911055516.5 and the article (Lacto-N-biose Synthesis via a Modular Enzymatic Cascade with ATP Regeneration. iScience 24 (3): 102236), 20 mmol/L galactose, 5 mmol/mL ATP, 10 mmol/L lacto-N-triose II, 20 U/mL galactokinase (Galk; EC2.7.1.6) , 10 mmol/L MgCl₂, 0.2 mg/ml Bgap enzyme, 2 U/mL acetate kinase (AcK; EC2.7.2.1), 10 U/mL pyruvate oxidase (PyoD; EC1.2.3.3) and 2 U/mL catalase (CAT; EC1.11.1.6) were dissolved in 100 mmol/L Tris-HCl buffer (pH 7.0) and the whole mixture was stirred at 30°C for 48 h. Finally, about 3.6 mmol/L of Lacto-N-tetraose (LNT) generated in this reaction was measured by HPLC analysis.

The above descriptions are only preferred examples of the present invention, and any equivalent changes and modifications made within the scope of the present invention patent application should fall within the scope of the present invention.

## Claims

1. An enzyme for the synthesis of lacto-N-tetraose, which belongs to the glycoside hydrolase family 112 when classified according to Carbohydrate-Active Enzymes, i.e., CAZy database.

2. The enzyme of the glycoside hydrolase family 112 according to claim 1, which is lacto-N-biose phosphorylase or 1,3-beta-galactosyl-N-acetylhexosamine phosphorylase.

3. The enzyme of the glycoside hydrolase family 112 according to claim 1 or 2, wherein the amino acid sequence of the lacto-N-biose phosphorylase is shown in SEQ ID NO:1, or the lacto-N-biose phosphorylase has an amino acid sequence of SEQ ID NO:1, or the enzyme of the glycoside hydrolase family 112 comprises lacto-N-biose phosphorylase with an amino acid sequence shown in SEQ ID NO:1; preferably, the enzyme of the glycoside hydrolase family 112 is derived from *Bacillus mycoides* (lnbp, Genebank No. QWJ02347.1) or *Bacillus cereus* (lnbp, Genebank No. WP_078422288.1).

4. The enzyme of the glycoside hydrolase family 112 according to claim 1 or 2, wherein the amino acid sequence of the 1,3-beta-galactosyl-N-acetylhexosamine phosphorylase is shown in SEQ ID NO:2, or the 1,3-beta-galactosyl-N-acetylhexosamine phosphorylase has an amino acid sequence of SEQ ID NO:2, or the enzyme of the glycoside hydrolase family 112 comprises 1,3-beta-galactosyl-N-acetylhexosamine phosphorylase with an amino acid sequence shown in SEQ ID NO:2; preferably, the enzyme of the glycoside hydrolase family 112 is derived from *Bifidobacterium longum* (gnpA, Genebank No. WP_131220594.1).

5. The enzyme of the glycoside hydrolase family 112 according to any one of claims 1-4, comprising an enzyme with an amino acid sequence having a sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, at least 100% to the amino acid sequence of SEQ ID NO:1, or SEQ ID NO:2.

6. A preparation method for the enzyme of the glycoside hydrolase family 112 according to any one of claims 1-5, wherein the enzyme of the glycoside hydrolase family 112 is generated by culturing a microorganism under the conditions enabling the growth of the microorganism;
preferably, the preparation method further comprises a step of recovering the generated enzyme;
preferably, the microorganism comprises wild bacteria or genetically engineered bacteria having an amino acid sequence shown in SEQ ID NO: 1 or SEQ ID NO:2, or having an amino acid sequence with a sequence identity of at least 70%, at least 75%, at least 80%, at least 85%, at least 90%, at least 95%, at least 97%, at least 98%, at least 99% or at least 100% to the amino acid sequence of SEQ ID NO:1 or SEQ ID NO:2;
preferably, the microorganism comprises *Bacillus mycoides, Bacillus cereus,* or *Bifidobacterium longum,* or genetically engineered bacteria of the above strains;
preferably, the host of the genetically engineered bacteria is any one selected from the group consisting of bacteria, fungi, and saccharomycetes;
preferably, the host of the genetically engineered bacteria is any one selected from the group consisting of *Escherichia coli, Vibrio natriegen, Bacillus subtilis, Bacillus licheniformis,* and *Bacillus αmyloliquefαcien;*
preferably, the host of the genetically engineered bacteria is any one selected from the group consisting of *Pichia pastoris, Saccharomyces cerevisiae, Kluyveromyces lactis,* and *Kluyveromyces marxianus;*
preferably, the host of the genetically engineered bacteria is any one selected from the group consisting of *Aspergillus oryzae, Aspergillus niger, Penicillium oxalicum,* and *Trichoderma reesei.*

7. Use of the enzyme according to any one of claims 1-5 in the synthesis of lacto-N-tetraose; preferably, the reaction for the synthesis of lacto-N-tetraose is conducted in an aqueous solution; preferably, the aqueous solution contains lacto-N-triose and galactose-1-phosphate and/or galactose and/or lactose.

8. The use of the enzyme of the glycoside hydrolase family 112 according to claim 7 in the synthesis of lacto-N-tetraose, wherein the enzyme of the glycoside hydrolase family 112 is a crude enzyme liquid or a purified enzyme liquid;
for the use, preferably, the enzyme of the glycoside hydrolase family 112 is one of the enzymes, or a combination thereof;
for the use, preferably, lacto-N-tetraose (LNT) is generated in an aqueous solution containing lactose and/or galactose and/or galactose-1-phosphate and lacto-N-triose (LNTII).

9. The use of the enzyme of the glycoside hydrolase family 112 according to claim 7 in the synthesis of lacto-N-tetraose, wherein a suitable temperature for the reaction is in the range of 10-70 °C; preferably, a suitable temperature for the reaction is in the range of 20-50 °C; more preferably, a suitable temperature for the reaction is in the range of 30-50 °C.

10. The use of the enzyme of the glycoside hydrolase family 112 according to claim 7 in the synthesis of lacto-N-tetraose, wherein lacto-N-tetraose is generated under a condition of pH 4-11; preferably, lacto-N-tetraose is generated under a condition of pH 6-8.

## Amended claims

### Amended claims under Art. 19.1 PCT

1. Use of an enzyme of the glycoside hydrolase family 112 for the synthesis of lacto-N-tetraose.

2. The use according to claim 1, wherein the amino acid sequence of the enzyme of the glycoside hydrolase family 112 is shown in SEQ ID NO:1 or SEQ ID NO:2.

3. The use according to claim 1, wherein the enzyme of the glycoside hydrolase family 112 is lacto-N-biose phosphorylase derived from *Bacillus mycoides* or *Bacillus cereus,* the amino acid sequence of which is shown in SEQ ID NO:1.

4. The use according to claim 1, wherein the enzyme of the glycoside hydrolase family 112 is 1,3-beta-galactosyl-N-acetylhexosamine phosphorylase derived from *Bifidobacterium longum,* the amino acid sequence of which is shown in SEQ ID NO:2.

5. The use according to claim 1, wherein the enzyme of the glycoside hydrolase family 112 is a crude enzyme liquid or a purified enzyme liquid.

6. The use according to claim 1, wherein the reaction for the synthesis of lacto-N-tetraose is conducted in an aqueous solution containing lacto-N-triose and galactose-1-phosphate and/or galactose and/or lactose.
